# EUROPEAN PATENT SPECIFICATION

(11) **EP 4 265 233 B1**
(45) Date of publication and mention of the grant of the patent: **14.10.2026**
(21) Application number: 23163912.1
(22) Date of filing: 24.03.2023
(51) Int. Cl.: A61F 13/56, A61F 13/58, A61F 13/15

(54) **AN ABSORBENT SANITARY ARTICLE WITH SIDE PANELS**
ABSORBIERENDER SANITÄRARTIKEL MIT SEITENTEILEN
ARTICLE HYGIÉNIQUE ABSORBANT AVEC PANNEAUX LATÉRAUX

(30) Priority: 22.04.2022 IT 202200007973
(43) Date of publication of application: 25.10.2023
(73) Proprietor: Fameccanica.Data S.p.A., 66020 San Giovanni Teatino (CH) (IT)
(72) Inventor: SABLONE, Gabriele, I-66020 San Giovanni Teatino (Chieti) (IT); GIANSANTE, Antonio, I-66020 San Giovanni Teatino (Chieti) (IT)
(74) Representative: Buzzi, Notaro & Antonielli d'Oulx S.p.A.

(56) References cited:
- EP-B1- 1 269 949
- WO-A1-2011/018167
- US-A1- 2008 319 412
- US-A1- 2015 202 091
- US-A1- 2019 365 582

## Description

### Field of the invention

The present invention relates to an absorbent sanitary article.

In particular, the invention relates to an absorbent sanitary article comprising a central body and at least one pair of side panels fixed to at least one end region of the central body.

The invention was developed in particular with a view to its application to absorbent sanitary articles for incontinent adults, in particular, for persons suffering from obesity.

### Technological background

Absorbent sanitary articles for incontinent adults generally comprise a central body or chassis with a rectangular shape elongated in a longitudinal direction and having a rear waist section, a front waist section and a crotch section extending between the rear waist section and the front waist section. The central body usually comprises an absorbent core sandwiched between a liquid-permeable topsheet which faces the wearer's skin in use, and a liquid-impermeable backsheet which faces the wearer's clothing in use. The central body may include an acquisition and distribution layer arranged between the topsheet and the absorbent core. The central body may also comprise a pair of elastic leg cuffs which extend along the topsheet in a longitudinal direction and which in use adhere to the user's legs.

Absorbent sanitary articles for incontinent adults generally comprise a pair of rear side panels and, optionally, a pair of front side panels. The rear side panels and, if provided, the front side panels have respective proximal sides fixed to corresponding side edges of the rear or front section of the central body and, in the condition of use, they extend laterally outwards with respect to the central body. The side panels are generally provided at the respective distal ends with closing elements for closing the sanitary article around the user's waist. The side panels may have inclined sides to conform to the user's legs. For example, the side panels may have a trapezoidal shape as described in EP-A-1941853.

Absorbent sanitary articles for obese adults are typically very large in size. For example, the central body may have a length of about one meter and a width of about 90 cm, so that the circumference of the waistband may reach dimensions in the order of 180 cm and above. In these cases, the side panels may have dimensions in the transversal direction that can reach 30 - 40 cm for each panel.

The large dimensions of the side panels pose various problems both from the point of view of the costs of the raw material and from the point of view of the production methods.

US20150202091A by the same applicant describes a method for producing absorbent sanitary articles wherein the side panels are partially overlapped and joined together in a provisional manner and are fixed to two side topsheet webs, which are subsequently joined to a central topsheet web.

EP1269949A describes a method for producing absorbent articles provided with side panels, which envisages longitudinally folding a continuous web in a generally Z-shaped configuration, separating single folded side panels from said Z-folded continuous web and attaching the folded side panels at predetermined intervals on a chassis tape.

US20190365582A1 discloses a disposable absorbent article comprising a chassis with first and second end portions and a central portion. The article includes a first and second pair of side panels folded over the chassis to form a folded configuration before use. In this configuration, the second panel is bonded to the chassis by a first breakable bond, and the first panel is bonded to the second panel by a second breakable bond in an overlapping area. The first breakable bond has a lower peel strength than the second.

US20150202091A1 discloses a method for producing pant-like sanitary articles with side panels. The method includes assembling a topsheet from a central fluid-permeable web and side materials, and partially overlapping and provisionally bonding first end regions of side panels to form a temporary laminate.

US20080319412A1 discloses a multi-configurable absorbent article with closure means that allows it to be worn in either a belt-type or slip-type configuration. Retractable belt portions are integrated along the rear panel, and releasable fasteners on the front panel can attach to the belt portions.

EP1269949A2 discloses a method for manufacturing a winged absorbent article. The method includes the steps of applying adhesive to side regions, folding wings, and applying fastening tabs in a manner that enhances structural integrity and fit.

WO201118167A1 discloses a folded incontinence article designed to be compact before use and easy to unfold during application. The article incorporates fold lines and positioning elements that maintain the article in a folded configuration until opened by the user.

### Object and summary

In the state of the art there remains the need to provide absorbent sanitary articles with large side panels which have lower costs of the raw material necessary for producing the side panels, and which can be produced with more efficient and economical equipment and production processes.

The object of the present invention is to provide an absorbent sanitary article that satisfies the aforesaid need and that solves the problems of the prior art.

According to the present invention, this object is achieved by an absorbent sanitary article having the characteristics of claim 1.

Preferred embodiments of the invention form the subject of the dependent claims.

The claims form an integral part of the technical disclosure provided in relation to the invention.

### Brief description of the drawings

The invention will now be described with reference to the attached drawings, given purely by way of nonlimiting example, wherein:
- Figure 1 is a plan schematic view of an absorbent sanitary article according to the present invention in an extended position,
- Figure 2 is a schematic cross-section along the line II-II of Figure 1.
- Figure 3 is a schematic cross-section illustrating a first embodiment of an absorbent sanitary article according to the present invention in an extended configuration,

- Figure 4 is a schematic cross-section illustrating a second embodiment of an absorbent sanitary article according to the present invention in an extended configuration, and
- Figures 5, 6 and 7 are schematic views illustrating various cross-sections of a method for producing absorbent sanitary articles according to the present invention.

### Detailed description

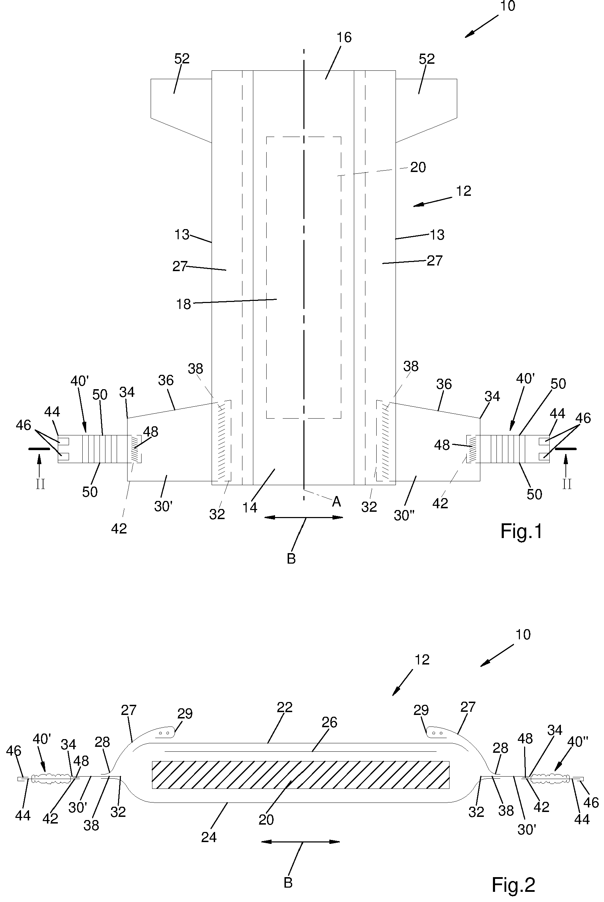

With reference to Figures 1-4, numeral 10 indicates an absorbent sanitary article according to the present invention. The absorbent sanitary article 10 comprises a central body 12 or chassis having an elongated shape along a longitudinal axis A. The central body 12 may have a rectangular shape, as illustrated in Figure 1 with two straight side edges 13 that are parallel to the longitudinal axis A. Alternatively, the two side edges 13 may be shaped to conform to the user's legs.

The central body 12 has a rear waist section 14, a front waist section 16 and a crotch section 18, which extends between the rear waist section 14 and the front waist section 16, and which in use is arranged between the legs of the user.

The central body 12 comprises an absorbent core 20 sandwiched between a liquid-permeable topsheet 22, which faces the wearer's skin in use and a liquid-impermeable backsheet 24 which faces the wearer's clothing in use. The central body 12 may include an acquisition and distribution layer 26 arranged between the topsheet 22 and the absorbent core 20.

The central body 12 may also comprise two leg cuffs 27 which extend longitudinally on the topsheet 22. Each leg cuff 27 comprises a non-woven strip having an outer edge 28 fixed to a corresponding side edge 13 of the central body 12 and an inner edge 29 in which at least one elastic thread tensioned in the longitudinal direction is arranged, so that in use the inner edge 29 of each leg cuff 27 adheres elastically against the user's legs.

The absorbent sanitary article 10 comprises first and second rear side panels 30', 30" which in the extended configuration of the absorbent sanitary article 10 extend in a transverse direction towards the outside with respect to the central body 12.

The first and second rear side panels 30', 30" have respective proximal edges 32 and respective distal edges 34. The first and second rear side panels 30', 30" may have transverse edges 36 shaped to conform to the user's legs in the condition of use. In possible embodiments, the rear side panels 30 may have a trapezium shape, in which the proximal edges 32 and the distal edges 34 form the major bases and the minor bases of the trapeziums, respectively.

The proximal edges 32 of the first and second rear side panels 30', 30" are fixed to corresponding side edges 13 of the rear waist section 14 of the central body 12. The attachment area between the proximal edges 32 of the first and second rear side panels 30', 30" and the side edges 13 of the rear section 14 is indicated by 38 in Figure 2.

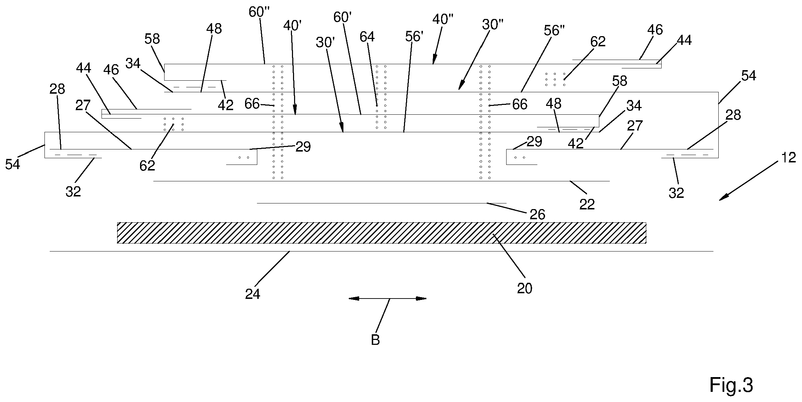

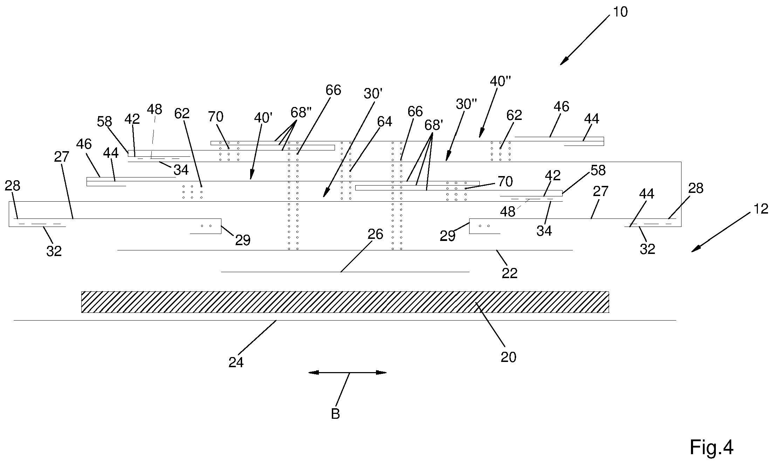

With reference to Figures 3 and 4, in a possible embodiment, the proximal edges 32 of the first and second rear side panels 30', 30" may be fixed between corresponding outer edges of the backsheet 24 and the outer edges 28 of the leg cuffs 27. The attachment may be carried out by welding (for example, thermo-compression or ultrasonic welding) or by glue.

In a possible embodiment, the proximal edges 32 of the first and second rear side panels 30', 30" may be fixed between corresponding outer edges of the backsheet 24 and of the topsheet 22.

The absorbent sanitary article 10 comprises first and second extension bands 40', 40" having respective proximal edges 42 fixed to respective distal edges 34 of respective first and second side panels 30', 30".

The fastening areas between the proximal edges 42 of the first and second extension bands 40', 40" and the respective distal edges 34 of the side panels 30', 30" are indicated by 48. The fastening of the first and second extension bands 40', 40" to the respective first and second side panels 30', 30" may be carried out by welding, such as, for example, thermo-compression or ultrasonic welding, or by glue.

In a possible embodiment, the first and second rear side panels 30', 30" and the first and second extension bands 40', 40" may be formed of different materials. In a possible embodiment, the first and second rear side panels 30', 30" may be formed of non-elastic material, for example, from one or more non-woven layers. In a possible embodiment, the first and second extension bands 40', 40" may be made of elastic material, extensible in a transverse direction B. Each extension band 40', 40" may be formed of an elastic laminate comprising at least one elastic element sandwiched between two non-woven layers. In a resting condition, each of the first and second extension bands 40', 40" may have pleated outer surfaces. The proximal edge 42 and the distal edge 44 of each extension band 40', 40" may be non-elastic.

Each extension band 40', 40" may have the shape of a strip elongated in a transverse direction, with two transverse sides 50 parallel to each other.

The closing elements 46 fixed to the distal ends 44 of the extension bands 40', 40" may be closing elements with micro-hooks or adhesives, which allow surface closure to be carried out with complementary closing elements to close the absorbent sanitary article 10 around the user's waist.

With reference to Figure 1, the absorbent sanitary article 10 may comprise a pair of front side panels 52, which in the extended configuration of the absorbent sanitary article 10, extend in a transverse direction towards the outside with respect to the central body 12.

The front side panels 52 may be fixed to corresponding side edges 13 of the front section 14 of the central body 12. The front side panels 52 may have outer surfaces configured to establish a surface closure with the closing elements 46. In case the absorbent sanitary article 10 is not provided with front side panels 52, the closing elements 46 may establish surface closure with an outer surface of the front section 14 of the central body 12. According to a further embodiment, both in the case wherein the absorbent sanitary article 10 is provided with front side panels 52, and in the case wherein the absorbent sanitary article 10 is not provided with front side panels 52, the absorbent sanitary article 10 may be provided with a front panel (frontal tape) fixed centrally on the outer surface of the absorbent sanitary article 10 facing outwards in use, or rather, towards the clothing. According to this further embodiment, the front panel is configured to establish a surface closure with the closing elements 46.

In the extended position, each of the extension bands 40', 40" may have a dimension at rest in the transversal direction B of between 100 and 400 mm.

The distance at rest in the transverse direction B between the distal edges 34 of the side panels 30', 30" may be between 600 and 900 mm. Consequently, the overall width at rest in the transverse direction B of the rear waistband of the absorbent sanitary article 10 may be between 800 and 1700 mm.

Figures 3 and 4 schematically illustrate two embodiments of an absorbent sanitary article 10 in folded positions. The folded positions illustrated in Figures 3 and 4 are the positions in which the absorbent sanitary articles 10 are found at the outlet of a production line and in the packages in which the absorbent sanitary articles 10 are marketed.

In both embodiments of Figures 3 and 4, the first and second side panels 30', 30" and the first and second extension bands 40', 40" are folded over the rear section 14 of the central body.

With reference to Figure 3, in the folded position, the first and second side panels 30', 30" are folded around respective first fold lines 54 with respect to the respective proximal edges 32 fixed to the central body 12, and have respective first and second panel flaps 56', 56".

The first and second extension bands 40', 40" are folded about respective second fold lines 58 with respect to the respective proximal edges 42 fixed to the respective first and second side panels 30', 30", and have respective first and second panel flaps 60', 60" overlapped on respective first and second panel flaps 56', 56".

The first panel flap 56' is overlapped on the topsheet 22 and the leg cuffs 27. The second panel flap 56" is overlapped on the first band flap 60'.

The first and second panel flaps 56', 56" and the first and second band flaps 60', 60" are held in the folded positions by temporary joining zones, which may be released with minimal peel force, and without damaging the elements between which they extend. Temporary bonds with these characteristics are well known in the absorbent sanitary article industry and may be formed by light welds (known in the art as "technical welds") or by small amounts of glue.

In a possible embodiment, first temporary joining zones 62 extend between the first and second band flaps 60', 60" and the respective first and second panel flaps 56', 56".

In a possible embodiment, at least one second temporary joining zone 64 extends between the second band flap 60" and the first panel flap 56', through the second panel flap 56" and through the first band flap 60'.

In a possible embodiment, at least one third temporary joining zone 66 extends between the second band flap 60" and the topsheet 22, through the second panel flap 56", through the first band flap 60' and through the first panel flap 56'. In the example illustrated in Figure 3, two third temporary joining zones 66 are provided, located on opposite sides with respect to the longitudinal axis A of the central body 12 of the absorbent sanitary article 10.

A second embodiment of an absorbent sanitary article in a folded configuration is illustrated in Figure 4. The elements corresponding to those previously described are indicated with the same numerical references. With respect to the embodiment of Figure 3, the first and second band flaps 60', 60" are further Z-folded and each of the band flaps 60', 60" has three flap sections 68', 68" overlapped on each other.

Also in the embodiment of Figure 4, the first and second panel flaps 56', 56", and the first and second band flaps 60', 60" are held in the folded positions by temporary joining zones 62, 64, 66 as previously described. In addition to the temporary joining zones 62, 64, 66 arranged as described in relation to Figure 3, in the embodiment of Figure 4 fourth temporary joining zones 70 may be provided, which extend between the first and second band flaps 60', 60" and the respective first and second panel flaps 56', 56" through the respective flap sections 68', 68" overlapped on each other.

It can be noted that in both embodiments of Figures 3 and 4, the closing elements 46 are uncovered and adjacent to the side edges 13 of the central body 12. To open the absorbent sanitary article in the position of use, it is sufficient to grasp the closing elements 46 and pull them in opposite directions outwards. In this way, the temporary joining zones 62, 64, 66, 68, which hold the first and second panel flaps 56', 56" and the first and second band flaps 60', 60" in the folded position yield and the side panels 30', 30" and the respective first and second extension bands 40', 40" are arranged in the open position shown in Figure 1.

Despite the considerable extension of the rear waistband in the open position (which can be between 800 and 1700 mm), in the folded configuration the absorbent sanitary article has a width equal to the width of the central body, which may be between 300 and 400 mm.

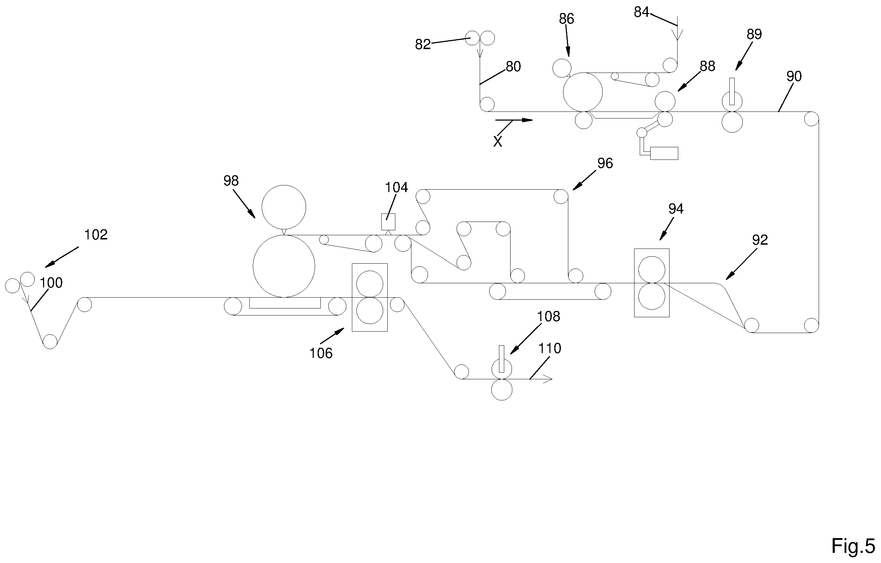

Figures 5, 6 and 7 schematically illustrate successive cross-sections of a method for producing absorbent sanitary articles 10 according to the present invention.

With reference to Figure 5, the method envisages feeding a continuous laminate 80 in the direction of its longitudinal axis X by a first feeding device 82. The continuous laminate 80 may be stretchable elastically in a transverse direction perpendicular to the longitudinal axis X.

A continuous closing tape 84, such as a micro-hook tape, is transversely cut in a first cut-and-slip unit 86 to form a row of closing elements (indicated by 46 in Figures 1-4). The first cut-and-slip unit 86 applies the closing elements along the side edges of the continuous laminate 80 in spaced apart positions along the longitudinal axis X. The closing elements may be fixed to the continuous laminate 80 in a first welding unit 88.

Then, the continuous laminate 80 is cut in the longitudinal direction in a first longitudinal cutting unit 89 to form two separate continuous webs 90, each of which has a row of closing elements at an outer edge.

The two continuous webs 90 are folded in the longitudinal direction in a longitudinal folding unit 92 so as to form on each of the two continuous webs 90 a single U-shaped lateral fold (embodiment of Figure 3) or a U-shaped lateral fold and a central Z-fold (embodiment of Figure 4).

After having made the folds, the folded edges of the two continuous webs 90 may be subjected to a compression step carried out in a compression unit 94.

After having made the folds, the two continuous webs 90 may be reversed in a first reversal unit 96, so that after the reversal the two continuous webs 90 have the respective closing elements along their respective edges facing each other.

Subsequently, the two continuous webs 90 are cut transversely in a second cut-and-slip unit 98 so as to form two rows of folded extension bands, indicated with 40', 40" in Figures 3 and 4.

The method envisages feeding a continuous web 100 by a second feeding unit 102. The continuous web 100 may be of non-elastic material, for example, a non-woven web. The second cut-and-slip unit 98 applies pairs of folded extension bands onto the continuous web 100 at positions spaced apart from each other in the longitudinal direction. The pairs of folded extension bands are fixed to the continuous web 100 along respective outer edges. This fastening may be carried out by glue, which can be applied to the two continuous webs 90 upstream of the second cut-and-slip unit 98 by a glue dispensing device 104. Alternatively, the pairs of folded extension bands may be fastened to the continuous web 100 by thermal or ultrasonic welding.

The pairs of folded extension bands fastened along their proximal edges to the continuous web 100 are then joined to the continuous web 100 in a temporary welding unit 106 so as to form the temporary joining zones indicated by 62 and 70 in Figures 3 and 4.

Then, the continuous web 100 is cut in the longitudinal direction in a second longitudinal cutting unit 108 so as to form two separate continuous webs 110, each of which has a respective row of folded extension bands fixed to one of its outer edges and joined to it by temporary joining zones.

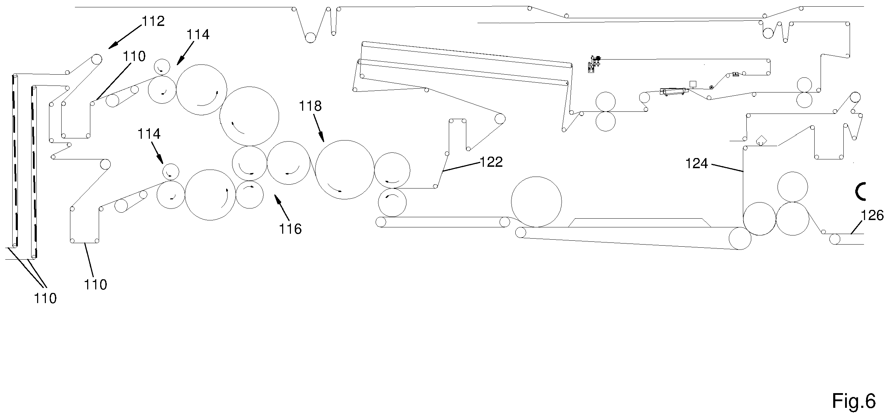

With reference to Figure 6, the two continuous webs 110 may be reversed in a second inversion unit 112, so that after the inversion the two continuous webs 110 have the edges along which the folded extension bands are fixed facing each other.

Then, the two continuous webs 110 are cut in respective cutting units 114 so as to form two rows of side panels, indicated with 30', 30" in Figures 1-4, each with a respective folded extension band 40', 40" fixed to one of its edges.

Then, each second side panel 30" with the respective second folded extension band 40" fixed thereto is partially overlapped on a first side panel 30', with the respective first folded extension band 40' fixed thereto. This overlapping may be done on an anvil wheel of a welding unit 116. In the welding unit 116, the first and second side panels 30', 30" and the respective folded extension bands 40', 40" fixed thereto and overlapped on each other may be subjected to welding operations to form the temporary joining zones 64 (Figures 3 and 4) that join together the second band flap 60" and the first panel flap 56' through the second panel flap 56" and through the first band flap 60'.

The row of overlapping side panels thus formed is fed to a repitching unit 118, which spaces the side panels apart in the longitudinal direction.

A leg cuff forming unit 120 forms two continuous strips of leg cuffs 122 provided with respective elastic bands and spaced apart from each other in the transverse direction.

The repitch unit 118 applies the row of side panels overlapped on each other on the two continuous strips of leg cuffs 122. The proximal edges of the first and second side panels 30', 30" may be glued or welded to the respective continuous strips of leg cuffs 122.

Then, a continuous topsheet web 124 is fixed between the two continuous strips of leg cuffs 122 to form a continuous composite web 126 including the continuous strips of leg cuffs 122, the continuous topsheet web 124 and a row of first and second side panels 30', 30" with respective folded extension bands 40', 40".

In a possible embodiment, the proximal edges of the first and second side panels 30', 30" may be fixed by glue or by welding to respective outer edges of the topsheet 22.

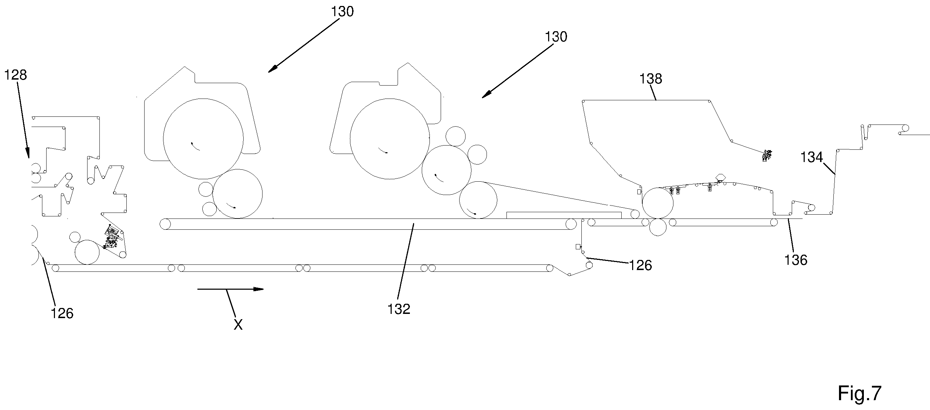

With reference to Figure 7, the continuous composite web 126 is made to advance in the X direction and acquisition and distribution layers may be applied thereon, indicated with 26 in Figures 1-4, formed by a unit 128.

At least one forming unit 130 forms absorbent cores, indicated by 20 in Figures 1-4, which advance spaced apart in the X direction on a conveyor 132. In the illustrated example, two forming units 130 are provided, which form two rows of overlapped absorbent cores.

The absorbent cores are sandwiched between the continuous composite web 126 and a continuous backsheet web 134 to form a continuous absorbent structure 136. Continuous leg elastic bands 136 may be applied between the continuous composite web 126 and the continuous backsheet web 134.

The side edges of the continuous strips of leg cuffs 122 may be secured to corresponding side edges of the continuous backsheet web 134 so as to fasten the proximal edges 32 of the side panels 30', 30" between the side edges of the continuous strips of leg cuffs 122 and the side edges of the continuous backsheet web 134.

In a possible embodiment, the side edges of the continuous strips of leg cuffs 122 may be fixed to corresponding side edges of the continuous topsheet web 124 and the side edges of the continuous topsheet 124 and backsheet 134 webs may be fixed to each other so as to fix the proximal edges 32 of the side panels 30', 30" between the side edges of the continuous topsheet 124 and backsheet 134 webs.

Then, the continuous absorbent structure 136 is cut to form individual absorbent sanitary articles 10.

Of course, without prejudice to the principle of the invention, the details of construction and the embodiments may vary, even significantly, with respect to those illustrated here, purely by way of nonlimiting example, without departing from the scope of the invention as defined by the attached claims.

## Claims

1. An absorbent sanitary article comprising:
- a central body (12) having a shape elongated along a longitudinal axis (A) and having a rear section (14), a front section (16) and a crotch section (18) extending between the rear section (14) and the front section (16), wherein the central body (12) comprises an absorbent core (20) sandwiched between a topsheet (22) and a backsheet (24),
- first and second side panels (30', 30") having respective proximal edges (32) fixed to corresponding side edges (13) of said rear section (14) or front section (16) of the central body (12), and respective distal edges (34), wherein in a folded configuration said first and second side panels (30', 30") are folded around respective first fold lines (54) with respect to the respective proximal edges (32) and have respective first and second panel flaps (56', 56"),
- first and second extension bands (40', 40") having respective proximal edges (42) fixed to respective distal edges (34) of respective first and second side panels (30', 30") and respective distal edges (44) to which at least one closing element (46) is fixed, wherein in said folded configuration said extension bands (40', 40") have respective band flaps (60', 60") overlapped to the respective panel flaps (56', 56"), wherein said band flaps (60', 60") are connected to the respective panel flaps (56', 56") by first temporary joining zones (62).

2. The absorbent sanitary article of claim 1, wherein the first panel flap (56') is overlapped to the topsheet (22) and the second panel flap (56") is overlapped to the first band flap (60').

3. The absorbent sanitary article of claim 2, comprising at least one second temporary joining zone (64) between the second band flap (60") and the first panel flap (56') extending through the second panel flap (56") and through the first band flap (60').

4. The absorbent sanitary article of any of claims 1-3, comprising at least one third temporary joining zone (66) between the second band flap (60") and the topsheet (22), extending through the second panel flap (56"), through the first band flap (60') and through the first panel flap (56').

5. The absorbent sanitary article of claim 1, wherein in said folded configuration said first and second extension bands (40', 40") are folded around respective second fold lines (58) with respect to the respective proximal edges (42).

6. The absorbent sanitary article of any of claims 1-5, wherein said first and second band flaps (60', 60") are further Z-folded and each of said first and second band flaps (60', 60") has three flap sections (68', 68") overlapped to each other.

7. The absorbent sanitary article of claim 6, comprising fourth temporary joining zones (70) between said first and second band flaps (60', 60") and the respective first and second panel flaps (56', 56"), extending through respective mutually overlapped flap sections (68', 68").

8. The absorbent sanitary article of any of the preceding claims, wherein said central body (12) comprises two leg cuffs (27) extending longitudinally on the topsheet (22) and wherein the proximal edges (32) of said first and second side panels (30', 30") are fixed between corresponding side edges of the backsheet (24) and of the leg cuffs (27).

9. The absorbent sanitary article of any of claims 1-7, wherein the proximal edges (32) of said first and second side panels (30', 30") are fixed between corresponding side edges of the backsheet (24) and of the topsheet (22).

## Patentansprüche

1. Absorbierender Hygieneartikel, umfassend:
- einen Mittelkörper (12) mit einer entlang einer Längsachse (A) langgestreckten Form und mit einem hinteren Abschnitt (14), einem vorderen Abschnitt (16) und einem Schrittabschnitt (18), der sich zwischen dem hinteren Abschnitt (14) und dem vorderen Abschnitt (16) erstreckt, wobei der Mittelkörper (12) einen saugfähigen Kern (20) umfasst, der zwischen einer Decklage (22) und einer Unterlage (24) angeordnet ist,
- erste und zweite Seitenpanele (30', 30") mit jeweiligen proximalen Kanten (32), die an entsprechenden Seitenkanten (13) des genannten hinteren Abschnitts (14) oder vorderen Abschnitts (16) des Mittelkörpers (12) befestigt sind, und mit jeweiligen distalen Kanten (34), wobei in einer gefalteten Konfiguration die genannten ersten und zweiten Seitenpanele (30', 30") um jeweilige erste Faltlinien (54) in Bezug auf die jeweiligen proximalen Kanten (32) gefaltet sind und jeweilige erste und zweite Panellaschen (56', 56") aufweisen,
- erste und zweite Verlängerungsbänder (40', 40") mit jeweiligen proximalen Kanten (42), die an jeweiligen distalen Kanten (34) der jeweiligen ersten und zweiten Seitenpanele (30', 30") befestigt sind, und mit jeweiligen distalen Kanten (44), an denen mindestens ein Verschlusselement (46) befestigt ist, wobei in der genannten gefalteten Konfiguration die genannten Verlängerungsbänder (40', 40") jeweilige Bandlaschen (60', 60") aufweisen, die mit den jeweiligen Panellaschen (56', 56") überlappen, wobei die genannten Bandlaschen (60', 60") mit den jeweiligen Panellaschen (56', 56") durch erste temporäre Verbindungszonen (62) verbunden sind.

2. Absorbierender Hygieneartikel nach Anspruch 1, wobei die erste Panellasche (56') mit der Decklage (22) überlappt und die zweite Panellasche (56") mit der ersten Bandlasche (60') überlappt.

3. Absorbierender Hygieneartikel nach Anspruch 2, umfassend mindestens eine zweite temporäre Verbindungszone (64) zwischen der zweiten Bandlasche (60") und der ersten Panellasche (56'), die sich durch die zweite Panellasche (56") und durch die erste Bandlasche (60') erstreckt.

4. Absorbierender Hygieneartikel nach einem der Ansprüche 1-3, umfassend mindestens eine dritte temporäre Verbindungszone (66) zwischen der zweiten Bandlasche (60") und der Decklage (22), die sich durch die zweite Panellasche (56"), durch die erste Bandlasche (60') und durch die erste Panellasche (56') erstreckt.

5. Absorbierender Hygieneartikel nach Anspruch 1, wobei in der genannten gefalteten Konfiguration die genannten ersten und zweiten Verlängerungsbänder (40', 40") um jeweilige zweite Faltlinien (58) in Bezug auf die jeweiligen proximalen Kanten (42) gefaltet sind.

6. Absorbierender Hygieneartikel nach einem der Ansprüche 1-5, wobei die genannten ersten und zweiten Bandlaschen (60', 60") weiter Z-gefaltet sind und jede der genannten ersten und zweiten Bandlaschen (60', 60") drei Laschenabschnitte (68', 68") aufweist, die miteinander überlappen.

7. Absorbierender Hygieneartikel nach Anspruch 6, umfassend vierte temporäre Verbindungszonen (70) zwischen den genannten ersten und zweiten Bandlaschen (60', 60") und den jeweiligen ersten und zweiten Panellaschen (56', 56"), die sich durch jeweilige gegenseitig überlappte Laschenabschnitte (68', 68") erstrecken.

8. Absorbierender Hygieneartikel nach einem der vorhergehenden Ansprüche, wobei der genannte Mittelkörper (12) zwei Beinbündchen (27) umfasst, die sich in Längsrichtung auf der Decklage (22) erstrecken, und wobei die proximalen Kanten (32) der genannten ersten und zweiten Seitenpanele (30', 30") zwischen entsprechenden Seitenkanten der Unterlage (24) und der Beinbündchen (27) befestigt sind.

9. Absorbierender Hygieneartikel nach einem der Ansprüche 1-7, wobei die proximalen Kanten (32) der genannten ersten und zweiten Seitenpanele (30', 30") zwischen entsprechenden Seitenkanten der Unterlage (24) und der Decklage (22) befestigt sind.

## Revendications

1. Article sanitaire absorbant comprenant :
- un corps central (12) ayant une forme allongée le long d'un axe longitudinal (A) et ayant une section arrière (14), une section avant (16) et une section d'entrejambe (18) s'étendant entre la section arrière (14) et la section avant (16), dans lequel le corps central (12) comprend une âme absorbante (20) prise en sandwich entre une feuille de dessus (22) et une feuille de fond (24),
- des premier et second panneaux latéraux (30', 30") ayant des bords proximaux respectifs (32) fixés à des bords latéraux correspondants (13) de ladite section arrière (14) ou section avant (16) du corps central (12), et des bords distaux respectifs (34), dans lequel dans une configuration pliée, lesdits premier et second panneaux latéraux (30', 30") sont pliés autour de premières lignes de pliage respectives (54) par rapport aux bords proximaux respectifs (32) et ont des premiers et seconds rabats de panneau respectifs (56', 56"),
- des première et seconde bandes d'extension (40', 40") ayant des bords proximaux respectifs (42) fixés à des bords distaux respectifs (34) des premier et second panneaux latéraux respectifs (30', 30") et des bords distaux respectifs (44) auxquels au moins un élément de fermeture (46) est fixé, dans lequel dans ladite configuration pliée, lesdites bandes d'extension (40', 40") ont des rabats de bande respectifs (60', 60") superposés aux rabats de panneau respectifs (56', 56"), dans lequel lesdits rabats de bande (60', 60") sont reliés aux rabats de panneau respectifs (56', 56") par des premières zones de jonction temporaires (62).

2. Article sanitaire absorbant selon la revendication 1, dans lequel le premier rabat de panneau (56') est superposé à la feuille de dessus (22) et le second rabat de panneau (56") est superposé au premier rabat de bande (60').

3. Article sanitaire absorbant selon la revendication 2, comprenant au moins une seconde zone de jonction temporaire (64) entre le second rabat de bande (60") et le premier rabat de panneau (56') s'étendant à travers le second rabat de panneau (56") et à travers le premier rabat de bande (60').

4. Article sanitaire absorbant selon l'une quelconque des revendications 1 à 3, comprenant au moins une troisième zone de jonction temporaire (66) entre le second rabat de bande (60") et la feuille de dessus (22), s'étendant à travers le second rabat de panneau (56"), à travers le premier rabat de bande (60') et à travers le premier rabat de panneau (56').

5. Article sanitaire absorbant selon la revendication 1, dans lequel dans ladite configuration pliée, lesdites première et seconde bandes d'extension (40', 40") sont pliées autour de secondes lignes de pliage respectives (58) par rapport aux bords proximaux respectifs (42).

6. Article sanitaire absorbant selon l'une quelconque des revendications 1 à 5, dans lequel lesdits premier et second rabats de bande (60', 60") sont en outre pliés en Z et chacun desdits premier et second rabats de bande (60', 60") a trois sections de rabat (68', 68") superposées les unes aux autres.

7. Article sanitaire absorbant selon la revendication 6, comprenant des quatrièmes zones de jonction temporaires (70) entre lesdits premier et second rabats de bande (60', 60") et les premier et second rabats de panneau respectifs (56', 56"), s'étendant à travers des sections de rabat mutuellement superposées respectives (68', 68").

8. Article sanitaire absorbant selon l'une quelconque des revendications précédentes, dans lequel ledit corps central (12) comprend deux manchettes de jambe (27) s'étendant longitudinalement sur la feuille de dessus (22) et dans lequel les bords proximaux (32) desdits premier et second panneaux latéraux (30', 30") sont fixés entre des bords latéraux correspondants de la feuille de fond (24) et des manchettes de jambe (27).

9. Article sanitaire absorbant selon l'une quelconque des revendications 1 à 7, dans lequel les bords proximaux (32) desdits premier et second panneaux latéraux (30', 30") sont fixés entre des bords latéraux correspondants de la feuille de fond (24) et de la feuille de dessus (22).
